# NEW EUROPEAN PATENT SPECIFICATION

(11) **EP 0 274 482 B2**
(45) Date of publication and mention of the opposition decision: **12.03.2003**
(45) Mention of the grant of the patent: 17.05.1995
(21) Application number: 87903043.5
(22) Date of filing: 26.03.1987
(51) Int. Cl.: C12Q 1/68

(54) **ISOLATED DNA SEQUENCES ASSOCIATED WITH MULTIDRUG RESISTANCE IN HUMAN CELLS**
MIT MEHRFACHARZNEIMITTELRESISTENZ ASSOZIIERTE,ISOLIERTE DNA-SEQUENZEN IN MENSCHLICHEN ZELLEN
SEQUENCES D'ADN ISOLEES ASSOCIEES A UNE RESISTANCE DES CELLULES HUMAINES A UNE MULTIPLICITE DE MEDICAMENTS

(30) Priority: 28.03.1986 US 845610; 01.08.1986 US 892575
(43) Date of publication of application: 20.07.1988
(73) Proprietor: THE BOARD OF TRUSTEES OF THE UNIVERSITY OF ILLINOIS, Urbana, Illinois 61801 (US)
(72) Inventor: RONINSON, Igor, B., Chicago, IL 60607 (US); PASTAN, Ira, H., Potomac, MD 20854 (US); GOTTESMAN, Michael, M., Bethesda, MD 20817 (US)
(74) Representative: Silveston, Judith
(86) International application number: US8700758
(87) International publication number: WO87005943

(56) References cited:
- PROCEEDINGS OF THE NATL. ACADEMY OF SCIENCES USA, vol. 82, no. 22, November 1985, Washington, DC (US); A.T. FOJO et al., pp. 7661, 7665#
- PROCEEDINGS OF THE NATL. ACADEMY OF SCIENCES USA, vol. 83, no. 2, January 1986, Washington, DC (US); P. CROSS et al., p. 337#
- CHEMICAL ABSTRACTS, vol. 105, October 1986, Columbus, OH (US); A. FOJO et al., p. 205, no. 128707q#
- JOURNAL OF CELLULAR BIOCHEMISTRY, vol. 29 (suppl.10A), no. 12, 1986; p. A18#
- JOURNAL OF CELLULAR BIOCHEMISTRY, vol. 29 (suppl. 10A), no. 9, 1986; p. A13#
- JOURNAL OF CELLULAR BIOCHEMISTRY, vol. 29 (suppl. 10A), no. 49, 1986; p. A130#
- NATURE, vol. 316, 29 August 1985; J.R. RIORDAN et al., pp. 817-819#
- NATURE, vol. 316, 29 August 1985; N. KARNTNER et al., pp. 820-823#
- CHEMICAL ABSTRACTS, vol. 106, no. 9, 02 March 1987, Columbus, OH (US); K. UEDA et al., p. 154, no. 62136q#
- CHEMICAL ABSTRACTS, vol. 105, no. 7, 18 August 1986, Columbus, OH (US); I.B. RONINSON et al., p. 165, no. 55593n#
- CHEMICAL ABSTRACTS, vol. 105, no. 3, 21 July 1986, Columbus, OH (US); J.R. RIORDAN et al., p. 226, no. 19855r#
- CHEMICAL ABSTRACTS, vol. 101, no. 7, 13 August 1984, Columbus, OH (US); V. LING et al., p. 145, no. 49442p#
- Second declaration of Dr I B Ronnson, Paras. 22-26, and Exhibit 1 filed therewith
- Abstracts A13, A18 and A130 of the UCLA Symposium on Molecular and Cellular Biology
- Somatic Cell and Molecular Genetics 11, 1985, p.117-126. Akiyama et al
- J.Biol.Chem. 261,1985,p.7762-7770.Shen et al
- Third declaration by Dr. I B Roninson.
- Second declaration of Dr I B Ronnson, Paras. 22-26, and Exhibit 1 filed therewith
- Abstracts A13, A18 and A130 of the UCLA Symposium on Molecular and Cellular Biology

## Description

### Background

The present invention pertains in general to diagnostic materials and methods and in particular to materials and methods for the detection of multidrug-resistant tumor cells..

Selection of mammalian cells for resistance to plant alkaloids or antitumor antibiotics frequently results in the development of cross-resistance to other drugs unrelated in their structure and mode of action to the original selective agent. Biedler et al., Cancer Res., 30, 1174 (1970). The phenomenon of multidrug resistance constitutes a major problem in cancer chemotherapy since it involves resistance to some of the most commonly used anticancer drugs.

Multidrug resistance in most cases appears to result from decreased intracellular drug accumulation, probably as a result of alterations in the plasma membrane. Biedler et al., Cancer Treat. Rep., 67, 859 (1983); Ling et al., Cancer Treat. Rep., 67, 869 (1983); Ramu et al., Cancer Treat. Rep., 67, 895 (1983); and Beck et al., Cancer Res., 39, 2070 (1979).

In some hamster, mouse and human multidrug-resistant cell lines, resistance correlates with over expression of a 170,000 m.w. membrane glycoprotein (P-glycoprotein) or a 19,000 m.w. cytosolic protein. Kartner et al., Science, 221, 1285-1288 (1983); Biedler et al., Cancer Treat. Rep., 67, 859 (1983). Immunoblotting techniques applied to cells from human cancer patients reveal high levels of P-glycoprotein in some cases of advanced, nonresponsive ovarian cancer. Bell et al., J. Clin. Oncol., 3, 311-315 (1985).

P-glycoprotein-specific, monoclonal antibodies raised against multidrug-resistant Chinese hamster ovary (CHO) cell lines and cross reactive with human cell lines apparently bind to multidrug-resistant mammalian cells to a degree correlated with the degree of their drug resistance. Kartner et al., Nature, 316, 820-823 (1985). These monoclonals may all bind to a C-terminal intracellular region of a proposed P-glycoprotein polypeptide. Kartner et al., Nature, 316, 820-823 (1985). P-glycoprotein specific cDNA clones have been isolated from Chinese hamster ovary cells, and these clones revealed amplification of the P-glycoprotein gene in multidrug resistant hamster, mouse and human cells when employed in a Southern blotting procedure. Riordan et al., Nature, 316, 817-819 (1985). However, Riordan et al. provides no indication whether the hamster P-glycoprotein cDNA clones may be used to detect the expression of human P-glycoprotein genes at the level of RNA.

In a different approach to the examination of multidrug-resistance, a common region of DNA is found to be amplified in two different multidrug-resistant Chinese hamster cell lines selected for resistance to either colchicine or Adriamycin. Roninson et al., Nature, 309, 626 (1984). This region was found to contain a transcription unit, presently designated mdr. Expression of the mdr mRNA correlates with multidrug resistance in the hamster cells. Gros et al., J. Cell. Biochem., 9C (suppl.), 16, AII67 (1985); and Gros et al., Proc. Natl. Acad. Sci. (USA), 83, 337 (1986). However, probes derived from the hamster mdr gene are not useful probes for human cells inasmuch as, even though these probes hybridize to human DNA (as illustrated in Example 2, infra), they do not hybridize efficiently with human mdr mRNA, despite the impression given in a report on a workshop dealing with multidrug resistance [Kolata, Science, 231, 220-221 (1986)].

Therefore, in the absence of a probe for human mdr gene expression, there is a need for a reliable method for detecting the presence of multidrug-resistant cells in a human tumor either prior to or during chemotherapy.

### Summary of the Invention

The present invention provides an isolated nucleic acid that forms a stable hybrid at 65°C in a solution consisting of 0.1 x SSC and 0.5% SDS with a polynucleotide having the sequence of nucleotides set forth in Table 5 or with the complement thereof, for example, with a polynucleotide having the sequence of nucleotides from nucleotide 1 to approximately nucleotide 3000 as set forth in Table 5 or with the complement thereof.

An isolated nucleic acid of the present invention is, for example, a nucleic acid having the sequence of nucleotides 425-4267 as set forth in Table 5.

An isolated nucleic acid of the present invention is obtainable as DNA or cDNA from a human cell.

The present invention also provides a nucleic acid probe, comprising a label and an isolated nucleic acid of the present invention.

### Brief Description of the Drawings

Fig. 1 is a partial restriction map of the cosmid clone cosDR3A which contains a 5' portion of the transcribed mdr region isolated from Chinese hamster cells;
Fig. 2 illustrates partial restriction maps of the plasmid clones pHDR4.4 and pHDR4.5, respectively containing mdr1 and mdr2 sequences; and
Fig. 3 illustrates partial restriction maps of phage cDNA clones λHDR5, λHDR10, λHDR62, λHDR28, λHDR69A, λHDR103, λHDR104 and λHDR105 containing mdr1 sequences.

### Detailed Description

Preliminary announcements of the obtaining of mdr1 clones according to the present invention and of uses therefor have been made by the inventors at the UCLA Symposia on Molecular and Cellular Biology, January 20 - February 15, 1986. Roninson et al., J. Cell. Biochem., 29 (suppl. 10A), 12, AI8 (1986); Pastan et al., J. Cell. Biochem., 29 (suppl. 10A), 9, AI3 (1986); Clark et al., J. Cell. Biochem., 29 (suppl. 10A), 49, AI30 (1986); and Cornwell et al., J. Cell. Biochem., 29 (suppl. 10A), 50, AI31 (1986).

A recently published European Patent Application No. 174,180 by John R. Riordan, entitled "Multidrug Resistance in Mammalian Cell Lines And Isolation Of Determinant Glycoprotein DNA," describes isolation of Chinese hamster cDNA clones specific for P-glycoprotein, and it suggests using P-glycoprotein-specific cDNA as a probe in determining multidrug resistance in cells. Although only Southern blot hybridization between hamster cDNA and human genomic DNA is described, claim 18 of Riordan, EPA 174,810, relates to a P-glycoprotein-specific DNA molecule "derived from a source selected from the group consisting of Chinese Hamster Ovary cells, mouse cells and human cells." In the event that the mdr clones described herein represent the human P-glycoprotein gene sequences, which is likely to be the case as discussed in Example 10 below, it should be noted that Riordan, EPA 174,810, does not disclose a human mdr gene or any portion thereof.

In fact, Riordan, EPA 174,810, post-dates the publication of Roninson et al., Nature, 309, 626 (1984) which described cloning of a segment of the Chinese hamster mdr region. The work describe in Roninson et al., Nature, 309, was followed by isolation of the entire Chinese hamster mdr gene [Gros et al., J. Cell. Biochem. and Proc. Nat'l. Acad. Sci. (USA), supra] as opposed to only partial cDNA clones of the Chinese hamster P-glycoprotein genes, as described in Riordan, EPA 174,810. Riordan, EPA 174,810, provides no evidence for the ability of Chinese hamster clones to detect the expression of human P-glycoprotein mRNA. Furthermore, the use of P-glycoprotein cDNA as a probe for detection of multidrug resistance in tumor cells is described in Riordan, EPA 174,180, only in terms of detection of amplified P-glycoprotein genes but not in terms of detection of increased P-glycoprotein mRNA expression. Increased mRNA expression, as described in Example 7 below, provides a much more useful diagnostic marker for multidrug resistance than does gene amplification. In addition, although claiming P-glycoprotein cDNA sequences of human origin, Riordan, EPA 174,810, contains no indication as to how such sequences would be obtained, e.g. the source of human DNA or RNA, or stringency conditions for screening of human cDNA or genomic libraries with a Chinese hamster probe. As shown in Example 2 below, there is a low level of homology between the hamster and human mdr genes, at least within the 5' half of the gene, which presents a considerable technical problem in the isolation of human mdr DNA sequences.

In the following examples, nucleic acid clones for human mdr genes and uses for the nucleotide sequences of mdr clones are described. In Example 1 a Chinese hamster mdr clone is used to identify sequences hybridizing with human DNA. Example 2 describes the identification and isolation of DNA sequences comprising human mdr genes. In Example 3, amplification of mdr genes in human drug-resistant cells is demonstrated. A characterization of clones containing mdr sequences is presented in Example 4. In Example 5, DNA rearrangement involving mdr genes is examined. In Example 6, transcription of the mdr1 gene in human cells is demonstrated. Example 7 describes an investigation into expression levels of the mdr1 sequence during the course of development of multidrug resistance in human cells. In Example 8, expression of mdr genes out of proportion to gene amplification is demonstrated. Example 9 provides a description of a genomic clone containing a segment of the mdr1 gene. In Example 10, cDNA clones of the mdr1 gene and the cDNA sequence of the human mdr1 gene is disclosed are described. In Example 11, diagnostic and therapeutic procedures using probes according to the present invention are described.

### Example 1

Derivation and characterization of multidrug-resistant sublines of human KB cells are described elsewhere. Akiyama et al., Somat. Cell Mol. Genet., 11, 117 (1985); Fojo et al., Cancer Res., 45, 3002 (1985); and Richert et al., Proc. Natl. Acad. Sci. (USA), 82, 2330 (1985). The multi-drug resistant phenotype is unstable in the most highly resistant lines, with a decrease in resistance when grown in the absence of the drugs. Using the in-gel DNA renaturation technique [according to Roninson, Nucleic Acids Res., 11, 5413 (1983)], several of the multidrug-resistant sublines of KB cells are known to contain amplified DNA sequences, and karyotypic analysis reveal double minute chromosomes in these cells. Fojo et al., Proc. Natl. Acad. Sci. (USA), 82, 7661 (1985).

Sublines of the human KB carcinoma cells, selected for resistance to colchicine, vinblastine or Adriamycin [Akiyama et al., supra; Fojo et al., Cancer Res. supra; Richert et al., supra and Shen et al., Science, 232, 643-645 (1986)], demonstrate the multidrug-resistant phenotype. Several of these sublines are described in Table 1. Fojo et al., Proc. Natl. Acad. Sci. USA, 82, 7661 (1985). In Table 1, "n/d" means not determined. KB-8-5-11, KB-8-5-11-24, KB-C3 and KB-C4 cell lines are subclones selected in 100 ng/ml, 1µg/ml, 3µg/ml and 4 µg/ml Adriamycin, respectively. Relative resistance is expressed as the D₁₀ of the resistant cell line divided by the D₁₀ of the parental KB-3-1 cells. Akiyama et al., supra.

**TABLE 1**

| Cell line | Relative Resistance To: | | |
|---|---|---|---|
| | Colchicine | Adriamycin | Vinblastine |
| KB-3-1 | 1 | 1 | 1 |
| KB-8-5-11 | 40 | 23 | 51 |
| KB-8-5-11-24 | 128 | 26 | 20 |
| KB-C3 | 487 | 141 | 206 |
| KB-C4 | 1750 | 254 | 159 |
| KB-C1-R1 | 6 | 3 | 4 |
| KB-V1 | 171 | 422 | 213 |
| KB-A1 | 19 | 97 | 43 |
| KB-A2 | n/d | 140 | n/d |

These multidrug-resistant human KB cell lines were used to determine whether DNA sequences homologous to the hamster mdr gene are present in the human genome. The Chinese hamster mdr DNA sequences used in this study were derived from the cosmid clone cosDR3A, containing a 5' segment of the hamster mdr gene. After digestion with the restriction enzymes XbaI and KpnI, individual 1.5 - 6 kilobase (kb) restriction fragments from this cosmid were either subcloned into pSP65 plasmid vector commercially available from Promega Biotec, Madison, Wisconsin, or gel-purified prior to labeling with ³²P. A vector including a 4.7 kb XbaI fragment, designated pDR4.7, contained DNA sequences hybridizing to human DNA.

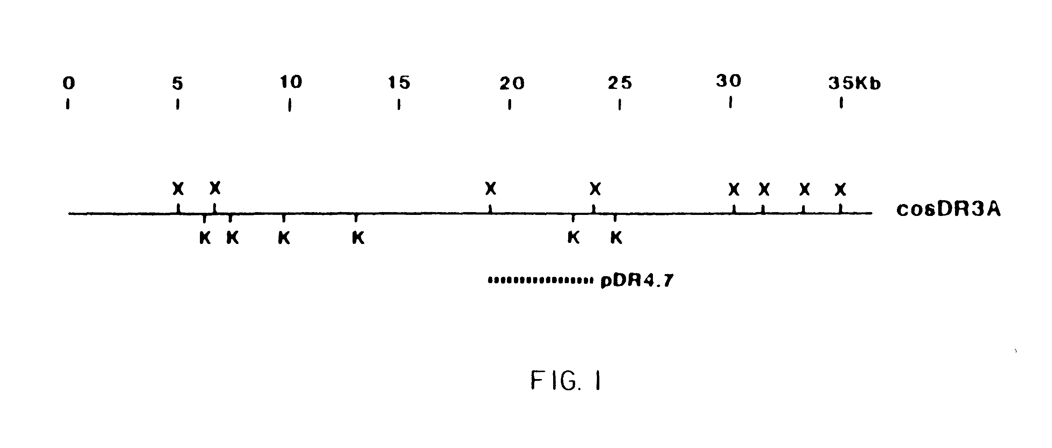

In Fig. 1, a partial restriction endonuclease map of the cosmid clone cosDR3A, containing a 5' portion of the transcribed mdr region amplified in multidrug-resistant Chinese hamster cells, is presented along with a dashed line aligned to indicate the portion of ROS DR3A which hybridizes to pDR4.7. In Fig. 1, X denotes an XbaI site and K identifies a KpnI site. Cloning and characterization of this region are described in Gros et al., Proc. Natl. Acad. Sci. USA, 83, 337 (1986).

### Example 2

In order to identify and isolate segments of DNA comprising the human mdr genes, individual 1.5 - 6 kilobase (kb) size fragments of the cloned hamster mdr gene were isolated as a series of recombinant subclones in a pSP64 plasmid vector commercially available from Promega Biotec and described in Promega Biotec Technical Bulletin No. 13 as well as in Melton, Nucleic Acids Res., 12, 7055-7056 (1984). Individual subclones were then labeled with ³²P and were used as probes for Southern blot hybridization with human DNA digested with restriction enzymes.

The subclones were then used as probes for hybridization with restriction digests of human genomic DNA. Most probes, when used under conditions of low hybridization stringency, produced either no hybridization signal or a continuous smear suggesting cross-hybridization with human repetitive DNA sequences. However, one of the subclones, designated pDR4.7 and illustrated in Fig. 1, gave rise to distinct bands when hybridized to human DNA under low stringency conditions.

Inasmuch as subclone pDR4.7, produced a distinct hybridization signal, this subclone contained hamster DNA sequences homologous to the human mdr genes. pDR4.7 hybridized to two major different EcoRI restriction fragments in human DNA, although in some experiments as many as nine additional EcoRI fragments could be detected.

### Example 3

In order to determine whether an mdr gene is amplified in multidrug-resistant human cells, DNA extracted from the parental KB-3-1 cells and various multidrug-resistant sublines described in Table 1 by the procedure of Gros-Bellard et al., Eur. J. Biochem., 36, 32 (1978) was digested with EcoRI or HindIII, electrophoresed on agarose gels and hybridized to the pDR4.7 probe by the procedure of Southern [Southern, J. Mol. Biol., 98, 503, (1975)].

In the Southern hybridization of pDR4.7 with EcoRI-digested DNA from multidrug-resistant KB cells, DNA was extracted as previously described [Gros-Bellard et al., Eur. J. Biochem., 36, 32 (1978)]. The concentration of EcoRI-digested DNA was determined by the diphenylamine reaction [Giles et al., Nature, 206, 93 (1965)] and 5 µg of DNA were loaded onto each lane. After electrophoresis, DNA was transferred onto a nylon (Biodyne) membrane [Southern, supra]. Plasmid pDR4.7 was digested with XbaI, the insert was gel-purified and labeled with ³²P to a specific activity of 3 x 10⁹ dpm/µg by oligolabeling [Feinberg et al., Analyt. Biochem., 132, 6 (1983)]. Hybridization was done at 65°C in 5 x SSPE, 5 x Denhardt's, 0.2% SDS, 500 µg/ml denatured salmon sperm DNA. After hybridization, the membranes were washed with 4 x SSC, 0.5% SDS at 65 ° C and autoradiographed.

The subclone pDR4.7 hybridizes to two EcoRI fragments of 13.5 and 4.5 kb size and to two HindIII fragments of 10.5 and 4.4 kb size in KB-3-1 DNA when the filters are washed under low stringency conditions (4 x SSC; 65°C). Only the 13.5 kb EcoRI and 4.4 kb HindIII fragments were detectable under conditions of intermediate stringency (1 x SSC; 65°C). All the fragments were amplified in colchicine-resistant sublines KB-8-5-11, KB-8-5-11-24, KB-C3 and KB-C4.

No amplification of either the band corresponding to the 13.5 kb fragment or the band corresponding to the 4.4 kb fragment was detected in the revertant subline KB-C1-R1. Unlike the colchicine-selected sublines, the subline KB-V1, selected in vinblastine, shows amplification of only the 13.5 kb EcoRI and the 4.4 kb HindIII bands. These two bands were also amplified in Adriamycin-resistant cells KB-A1 and KB-A2. KB-A1, in addition, contained a new amplified band of a 7 kb size in the EcoRI digest and of a 6.5 kb size in the HindIII digest. The same bands were present in KB-V1 DNA, but their intensity suggested that these bands were not amplified. No bands of this size were detected in the parental KB-3-1 DNA, suggesting that they apparently arose as a result of a DNA rearrangement.

The different patterns of amplification of the two types of bands hybridizing to the hamster mdr probe in different sublines suggested that they might correspond to two different related DNA sequences, possibly different members of a multigene family, rather than to two different parts of one contiguous hybridizing region. DNA sequences corresponding to the 13.5 kb EcoRI and 4.4 kb HindIII fragments were designated mdr1 and the sequences corresponding to the 4.5 kb EcoRI and the 10.5 kb HindIII fragments were designated mdr2.

The degree of amplification of mdr sequences in different multidrug-resistant sublines was estimated by comparing the intensity of hybridization signals from serially diluted EcoRI digests of different cellular DNAs. The estimates of the copy number of mdr sequences in different sublines are given in Table 2. In Table 2, a star indicates the rearrangement of mdr2 DNA sequences.

**TABLE 2**

| Cell Line | Degree of Amplification | |
|---|---|---|
| | mdr1 | mdr2 |
| KB-3-1 | 1 | 1 |
| KB-8-5-11 | 7-8 | 7-8 |
| KB-8-5-11-24 | 9 | 9 |
| KB-C3 | 20 | 20 |
| KB-C4 | 30 | 30 |
| KB-C1-R1 | 1 | 1 |
| KB-V1 | 100 | 1* |
| KB-A1 | 70 | 30* |
| KB-A2 | 80 | 1 |

By comparison of Table 1 with Table 2, it may be observed that in the sublines selected for a 40-700 fold degree of resistance to colchicine, there is a general, but not precise, correlation between increases in drug resistance and in the copy number of mdr sequences. The degree of resistance may correlate more precisely with the expression of mdr RNA than with the degree of mdr gene amplification. The mdr1 and mdr2 sequences appear to be amplified to a similar degree in these cells. The loss of amplified mdr sequences in a revertant of a colchicine-resistant cell line provides strong additional evidence that mdr gene amplification underlies multidrug resistance in the highly resistant cells. The degree of amplification of mdr1 in the cells selected for resistance to vinblastine or Adriamycin appears to be higher than in the cells with a similar degree of resistance that have been selected with colchicine.

### Example 4

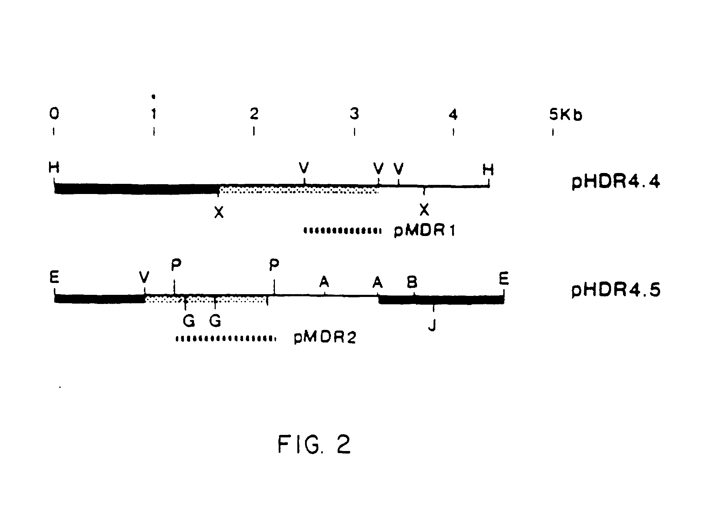

To investigate the nature of the human mdr genes, clones containing mdr1 and mdr2 sequences were isolated from the DNA of the colchicine-resistant subline KB-C3. For this purpose, two phage libraries containing complete EcoRI or HindIII digests of KB-C3 DNA were prepared. The EcoRI library was constructed by insertion into the EcoRI site of the λgtII phage vector, and the HindIII library was made by insertion into the HindIII site of Charon 28 [Young et al., Proc. Natl. Acad. Sci. (USA), 80, 1194 (1983); Rimm et al., Gene, 12, 301 (1980)]. Both libraries were screened by plaque hybridization with the Chinese hamster pDR4.7 probe according to the procedure of Benton et al., Science, 196, 180 (1977). A clone containing the 4.4 kb HindIII fragment (mdr1) was isolated from the HindIII library, and a clone containing the 4.5 kb EcoRI fragment (mdr2) was isolated from the EcoRI library. Both inserts were subsequently recloned into the plasmid vector pSP64 [Melton et al., Nucleic Acids Res., 12, 7035 (1984)], giving rise to plasmid clones designated pHDR4.4 and pHDR4.5, respectively. Plasmid clone pHDR4.4 was deposited with the American Type Culture Collection, 12301 Parklawn Drive, Rockville, Maryland, as Deposit No. 40227 on March 21, 1986. Likewise, plasmid clone pHDR4.5 was deposited with the American Type Culture Collection, 12301 Parklawn Drive, Rockville, Maryland, as Deposit No. 40228 on March 21, 1986. Partial restriction maps of these clones are shown in Fig. 2. In Fig. 2, sites for digestion by corresponding restriction endonucleases are identified as follows: "A", AvaI; "B", BamHI; "E", EcoRI; "G", BgIII; "H", HindIII; "J", HaeII; "P", PstI; "V", PvuII; and "X", XbaI.

In Fig. 2, solid bars indicate the fragments containing highly repeated sequences. These fragments were identified by hybridization of Southern blots containing restriction digests of cloned DNA with 0.35 x 10⁵ dpm/cm² of ³²P-labeled total human genomic DNA. Dashed lines indicate the DNA sequences hybridizing to the pDR4.7 clone, as determined by Southern hybridization with the gel-purified pDR4.7 insert.

Because the pDR4.7 hamster probe was known to contain transcriptionally active sequences expressed in multidrug-resistant hamster cells [Gros et al., Proc. Nat'l. Acad. Sci. (USA), supra] it seemed likely that the conserved human mdr sequences would provide convenient probes for transcription studies. The hamster pDR4.7 probe hybridized very poorly, if at all, to mRNA from multidrug-resistant human cells, and therefore could not be used as a probe for detection of mdr genes in human cells. Consequently, repeat-free fragments of both clones which hybridized to pDR4.7 were subcloned into the plasmid vector pSP64. The clone containing a 0.75 kb PvuII fragment of pHDR4.4, inserted into the SmaI site of the vector, was designated pMDR1. The clone containing a 1.0 kb Pstl fragment of pHDR4.5, inserted into the PstI site of the vector, was designated pMDR2. These two clones were found to cross-hybridize with each other under conditions of low hybridization stringency providing additional evidence that mdr1 and mdr2 represent related DNA sequences.

### Example 5

To determine whether the rearranged bands in KB-V1 and KB-A1 correspond to mdr1 or mdr2, DNA from different sublines was digested with HindIII and hybridized to either hamster pDR4.7 probe or to the human pMDR1 or pMDR2 probes. Hybridization with the gel-purified insert of the plasmid pDR4.7 was done under conditions of low stringency (4 X SSC, 0.5% SDS at 65°C). The same blot was then rehybridized with gel-purified inserts of the plasmids pMDR1 and pMDR2 under high stringency conditions (0.1 X SSC, 0.5% SDS at 65°C) so that the signal resulting from cross-hybridization of mdr1 and mdr2 sequences was minimized.

This experiment demonstrated that rearranged bands in both KB-A1 and KB-V1 sublines correspond to mdr2. The mobility of the new bands appears to be identical in several different restriction digests of KB-V1 and KB-A1 DNA, indicating that a similar rearrangement may have occurred in both independently selected sublines. However, while the rearranged bands are amplified in KB-A1, they do not appear amplified in KB-V1 cells. In addition, both types of cells contain bands corresponding to the unrearranged allele of mdr2, which is not amplified. Amplification of the rearranged but not the parental mdr2 band in KB-A1 cells suggests that DNA rearrangement either preceded or occurred simultaneously with the onset of gene amplification in these cells. In the case of KB-V1 it is unclear whether mdr2 rearrangement is related to amplification of mdr1.

### Example 6

To determine whether the evolutionarily conserved regions of mdr1 and mdr2 contained transcribed sequences, pMDR1 and pMDR2 were used as probes for Northern hybridization, performed according to the procedure of Thomas, Proc. Natl. Acad. Sci. (USA), 77, 5201-5205 (1980) with poly (A)⁺ RNA extracted from the parental KB-3-1 and multidrug-resistant KB-C2.5 cells [Akiyama et al., supra; Fojo et al., Cancer Res., supra; Richert et al., supra and Shen et al., supra] under the conditions of high hybridization stringency as recited in Example 5. Poly (A)⁺ RNA was extracted from the parental drug-sensitive KB-3-1 cells and from the colchicine-resistant KB-C2.5 subline as described in Chirgwin et al., Biochem., 18, 5294 (1979). One microgram of each RNA preparation was electrophoresed in a 1.5% glyoxal agarose gel [McMaster et al., Proc. Natl. Acad. Sci. (USA), 74, 4835 (1977)] and transferred onto Gene Screen Plus™ membrane as available from New England Nuclear, Boston, MA. The membranes were hybridized with 3 x 10⁵ dpm/cm² of pMDR1 or pMDR2 probes. Hybridization was done in 1M NaCI, 10% dextran sulphate, 1% SDS, 50% formamide, 100 µg/ml denatured salmon sperm DNA at 42°C. The membranes were washed with 0.1 x SSC, 0.5% SDS at 65°C and autoradiographed. The size of the RNA band was determined relative to the positions of 28S and 18S ribosomal RNA.

The probe pMDR1 hybridizes to an mRNA band of a 4.5 kb size which is highly expressed in the drug-resistant cells. This mRNA is not detectable in the parental KB-3-1 cells, indicating little or no expression when the probes were labelled either by nick translation or oliogolabelling. No distinct bands, however, could be detected when pMDR2 was used as a probe. In addition, no bands were revealed by using other repeat-free subfragments of pHDR4.5 as probes in addition to pMDR2. While the existence of transcriptionally active sequences in other regions of mdr2 or transcription of mdr2 sequences at a very low level cannot be excluded by these results, transcription of the amplified region of mdr2 homologous to the Chinese hamster mdr gene is not detected by Northern hybridization.

Amplification and over expression of DNA sequences homologous to the Chinese hamster mdr gene in multidrug-resistant human KB carcinoma cells suggests that a similar mechanism may be responsible for multi-drug resistance in both human and rodent cells. The nature of the proteins encoded by mdr genes is still unknown. The size of mdr1 mRNA is consistent with the possibility that it may code for a 170 kd glycoprotein overexpressed in various multidrug-resistant cell lines [Biedler et al., supra; Ling et al., supra; Ramu et al., supra; Beck et al., supra; Kartner et al., Science, 221, 1285 (1983); Debenham et al., Mol. Cell. Biol., 2, 881 (1982); Robertson et al., Mol. Cell. Biol., 4, 500 (1984)]. It is also unknown whether the same mechanism is utilized in the development of multidrug resistance by human tumor cells in vitro and in the course of chemotherapy. The availability of cloned probes which detect transcription of mdr1 DNA in human cells makes it possible now to investigate expression of these sequences in clinical samples of multidrug-resistant tumors.

### Example 7

In order to examine levels of expression of mdr1 sequences during the development of multidrug resistance, multidrug-resistant sublines of human KB carcinoma cells and two other human multidrug resistant cell lines of different origin were studied.

Agents used in selecting different sublines in multiple steps were colchicine, Adriamycin and vinblastine. In the first two steps of colchicine selection, clones were only obtained if the cell populations were first mutagenized with ethylmethane sulfonate (EMS). Similarly, KB cell lines selected independently for resistance to Adriamycin or vinblastine [Akiyama et al., supra; Fojo et al., Cancer Res., supra; Richert et al., supra; and Shen et al., supra] were obtained only after mutagenesis with EMS in the first step. Subsequent selection, up to very high levels of resistance, was possible without mutagenesis, and occurred at high frequency.

The isolation and some properties of the human multidrug resistant KB carcinoma cell lines has been previously described in Akiyama et al., supra; Fojo et al., Cancer Res., supra; and Richert et al., supra. The KB cell lines used in this study, the manner of their selection, and their relative resistance to various drugs, are shown in Table 3. CEM is a cell line described in Beck in Advances in Enzyme Regulation, 22, G. Weber, ed. (Pergamon Press, Oxford, 1984), 207, and 2780 is a cell line described in Rogan et al., Science, 224, 994 (1984).

To determine the extent to which mdr1 sequences were expressed in these cell lines and the size of the corresponding RNAs, a Northern hybridization was performed with total RNA and poly (A)⁺-RNA from these cells. A 4.5 kilobase RNA, which migrates just below the 28S ribosomal RNA marker, was clearly visible in all the lanes containing either total or poly (A)⁺ RNA from the resistant lines but was not seen in any of the sensitive cell lines.

Slot blot hybridization of total RNA was used to quantitate the. expression of mdr1 in various sensitive and resistant cell lines. RNA prepared as previously described above was applied to filters using a Schleicher and Schuell slot blot apparatus or by blotting after electrophorosis in 1% agarose containing 13.4% formaldehyde. A gel-purified insert from the pMDR1 clone was ³²P-labeled for use as a probe. Nitrocellulose filters were baked and preincubated for 4-6 hours at 42°C in 50% formamide, 5 x SSC, 10X Denhardt's solution, 0.1% SDS and 100 µg/ml salmon sperm DNA. Filters were hybridized overnight in the above solution containing ³²P-labeled probe. Filters were washed 3 times for 10 minutes at room temperature in 2 x SSC, 0.1% SDS and 3 times for 20 minutes at 50°C in 0.1 x SSC, 0.1% SDS. Levels of mdr1 expression were determined by densitometry of the autoradiograms. Tracings of peaks were cut out and weighed and compared to the KB-8 peak which was arbitrarily assigned a value of 1. The results are presented in Table 3 along with the relative drug resistances of the human leukemic lymphoblast cell lines, and the human ovarian cancer cell lines used in the study. In Table 3, ND is an abbreviation for "none detected".

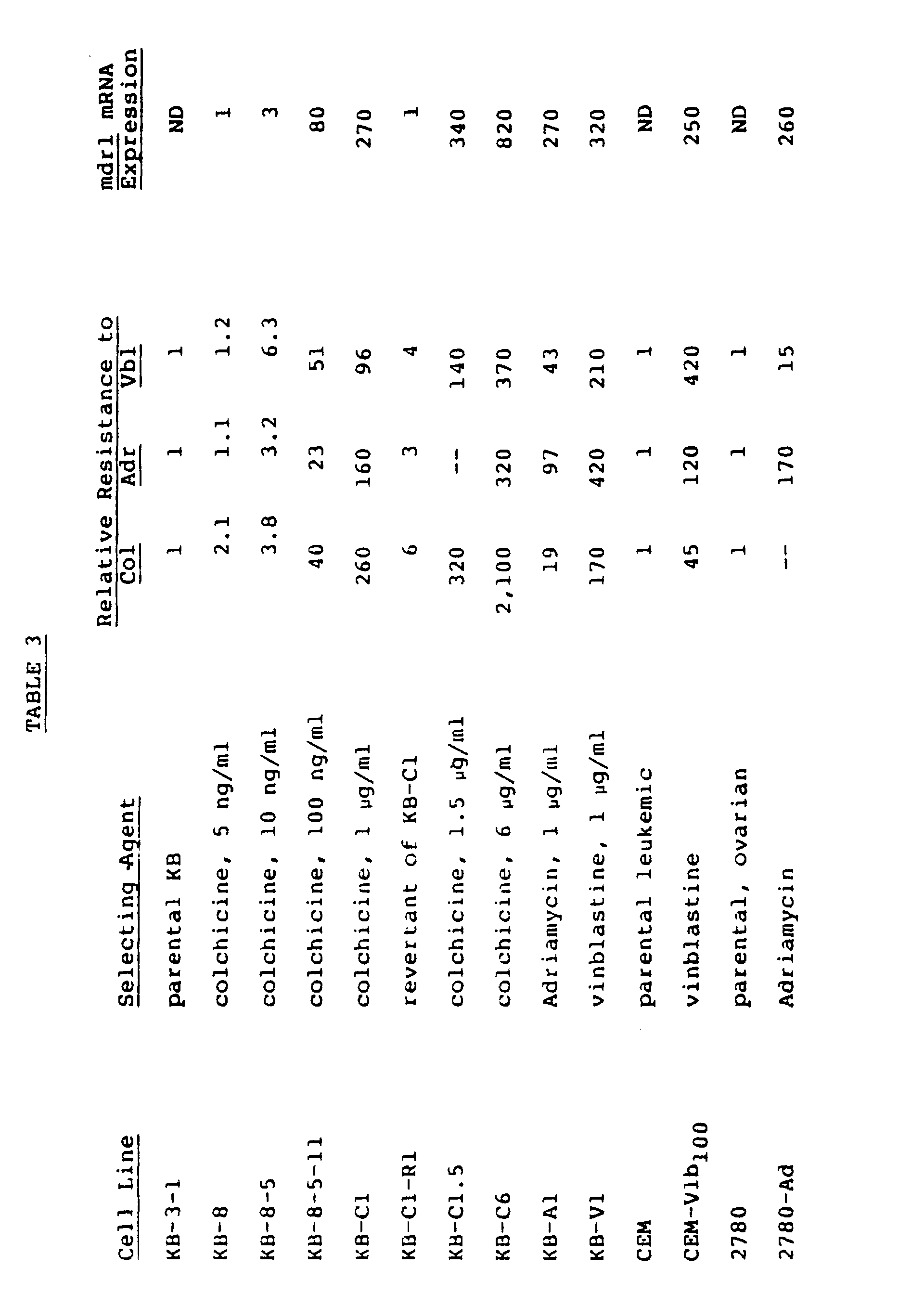

As shown in Table 3, there was a good correlation between extent of multidrug resistance and the level of mdr1-specific mRNA. As can also be seen in Table 3, there is little or no expression of the mdr1 sequences in parental, drug-sensitive cell lines, but increasing expression occurs as the cell lines become more resistant to drugs. A revertant cell line, KB-C1-R1, subcloned in the absence of colchicine from the colchicine-resistant cell line KB-C1, still expresses mdr1 sequences at reduced levels consistent with its low level of multidrug resistance.

It is not possible to calculate the exact extent of increased expression in the resistant cell lines relative to the parental line, since the hybridization signal from the parental RNA was too weak. However, the extent of expression relative to the KB-8 cell line has been calculated and these data are shown in Table 3. Expression appears to correlate well with increasing drug-resistance for every step of selection in KB cells and reaches very high levels in our most resistant KB cell lines.

The data summarized in Table 3 indicate that two other human cell lines of different origin, selected for multidrug resistance, also express high levels of the 4.5 kb mRNA. Very little or no expression of this RNA was detected in the parental cell lines. The human leukemic lymphoblast cell line CEM (A.T.C.C. CCLII9) and its resistant derivatives CEM-VLB₁₀₀, selected for resistance to vinblastine (gift of W. Beck, St. Jude's Hospital) (Beck, supra.) and the ovarian cell line 2780 and its resistant derivative 2780-Ad, selected for resistance to Adriamycin (gift of T. Hamilton and R. Ozols, National Institutes of Health) (Rogan et al., supra) both showed high levels of expression of the 4.5 kb mRNA. Because even low levels of cellular multidrug-resistance may result in clinically refractory tumors, expression of mdr1 mRNA in sublines having a low level (2-6 fold) of relative drug resistance but not in the parental drug-sensitive cell lines is of particular interest. In this regard the results presented in Table 3 indicate that quantitation of mdr1 mRNA expression may potentially be used for diagnosis of multidrug resistance in clinical tumor specimens.

### Example 8

To compare the levels of mdr1 mRNA expression with the extent of amplification of the genomic mdr1 sequences genomic DNA was isolated from all of the cell lines described in Example 6. Following digestion with HindIII, amplification of mdr1 was examined by Southern blot analysis.

DNA, prepared as previously described in Example 3, was digested with HindIII and electrophoresed in 0.8% agarose gels before Southern transfer to Gene Screen Plus™ (New England Nuclear). The blots were hybridized with the pMDR1 probe for 18 hours at 42°C in 50% formamide, 5 x SSC, 1% SDS with 100 µg/ml salmon sperm DNA. The blots were then washed with 2 x SSC at room temperature for 10 minutes, 2 x SSC, 1% SDS at 42°C for 60 minutes and 0.1 x SSC at room temperature for 60 minutes prior to autoradiography.

No amplification of mdr1 was found in the KB cell lines with low levels of resistance (KB-8, KB-8-5 and the revertant subline, KB-C1-R1), even though these cell lines expressed increased levels of mdr1 mRNA. Increased expression of mdr1 sequences in human cells may therefore occur prior to gene amplification. Amplification of the mdr1 gene was detected in highly resistant sublines of KB cells selected in colchicine, vinblastine or Adriamycin, as well as in CEM-VLB₁₀₀ and 2780-Ad cell lines. In the latter two sublines, the degree of gene amplification was estimated by densitometry to be approximately 5-10 fold for 2780-Ad and 10-15 fold for CEM-VLB₁₀₀.

In all cases, the increase in mRNA expression was clearly greater than the extent of amplification. These results suggest that the evolution of these lines involved a step or steps in which expression was increased out of proportion to gene amplification. A similar dissociation of amplification and expression of the dhfr gene has been reported for human cancer cells selected for resistance to methotrexate in vitro. [Frei et al., Proc. Natl. Acad. Sci. (USA), 81, 2873 (1984); Wolman et al., Proc. Natl. Acad. Sci. (USA), 80, 807 (1983).] The development of multidrug resistance in human KB cells differs in this respect from Chinese hamster V79 cells where a low (5-7 fold) degree of relative drug resistance is accompanied by 5-10 fold amplification of mdr DNA [Roninson et al., supra; and Gros et al., supra].

These studies demonstrate a correlation between expression of the mdr1 gene and the development of resistance to multiple agents in five independently-derived human cell lines of different origins selected for resistance to different cytotoxic drugs. Expression of mdr1 may therefore represent a common mechanism of multidrug resistance in human cell lines. Increased expression of mdr1 in at least some cases occurs initially without gene amplification and may be a prerequisite for the development of multidrug resistance. This observation may be especially relevant for the analysis of the role of the mdr1 gene in the development of multidrug resistance by human tumors in the course of chemotherapy and may have diagnostic potential. Since the tumor calls are expected to have a relatively low degree of resistance, such an analysis may involve quantitation of mdr1 RNA expression rather than gene amplification in tumor samples.

### Example 9

The segment of the mdr1 gene cloned into pMDR1 was sequenced by the chemical degradation procedure [Maxam et al., Meth. Enzymol., 65, 499, (1980)] and the enzymatic chain-termination sequencing technique [Sanger et al., Proc. Natl. Acad. Sci. USA, 74, 5463, (1977)] using supercoiled plasmid DNA as a template [Zagursky et al., Gene Anal. Techn., 2, 89, (1985)]. To facilitate sequencing, pMDR1 was mapped with HaeIII and RsaI and individual 220-400 bp fragments of pMDR1 were subcloned into a pUCI8 plasmid vector (Bethesda Research Laboratories, Rockville, MD). The sequence of pMDR1 was confirmed by sequencing both strands. The complete sequence of pMDR1 is presented in Table 4. Comparison with the sequence of the corresponding cDNA clones in Example 10 below indicated that pMDR1 includes segments of two protein-coding sequences (exons), comprising nucleotides 1-111 and 653-807, and an intervening sequence (intron) which is not expressed as mRNA and which comprises nucleotides 112-652. Table 4 shows that amino acid sequence corresponding to the exons within pMDR1. This amino acid sequence therefore defines a segment of the mdr1 protein product.

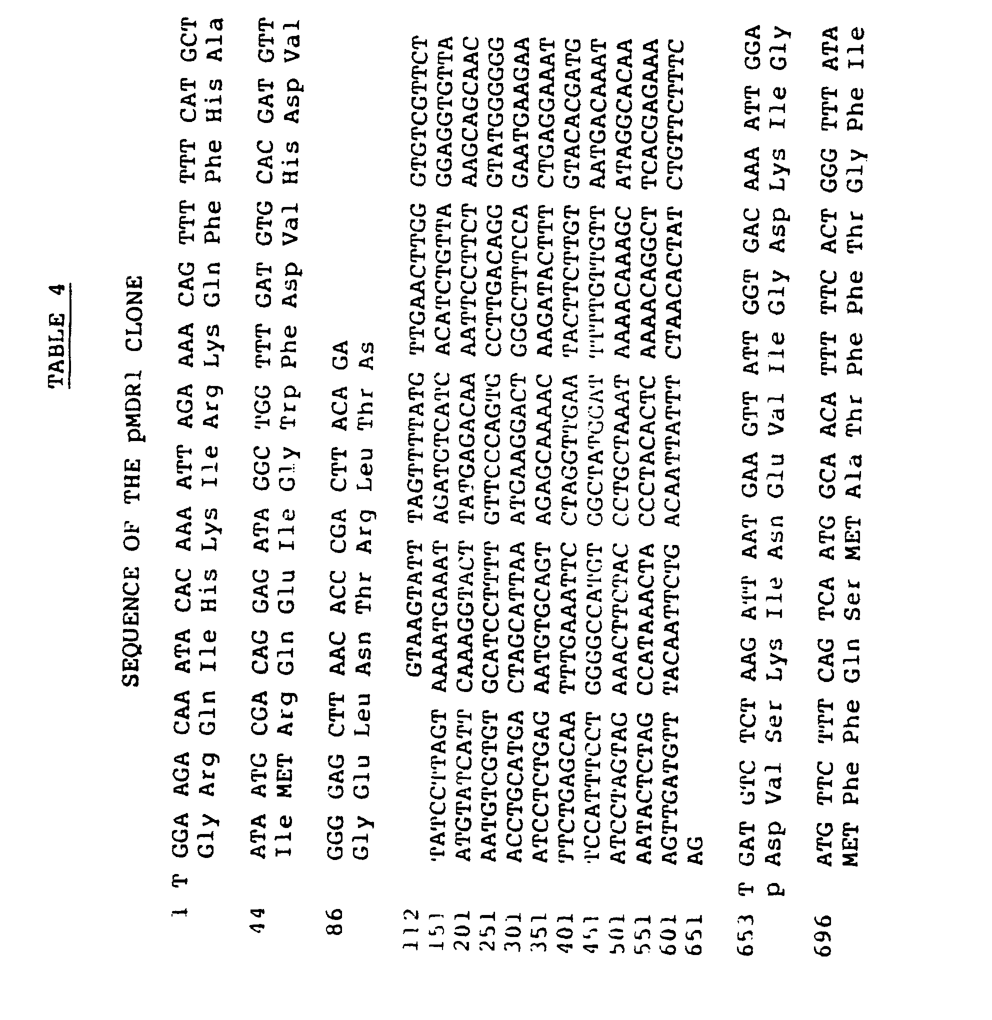

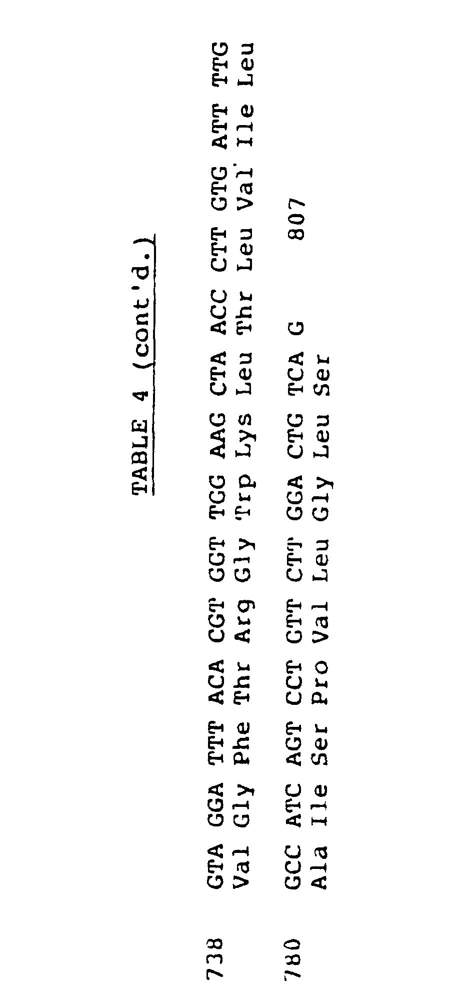

### Example 10

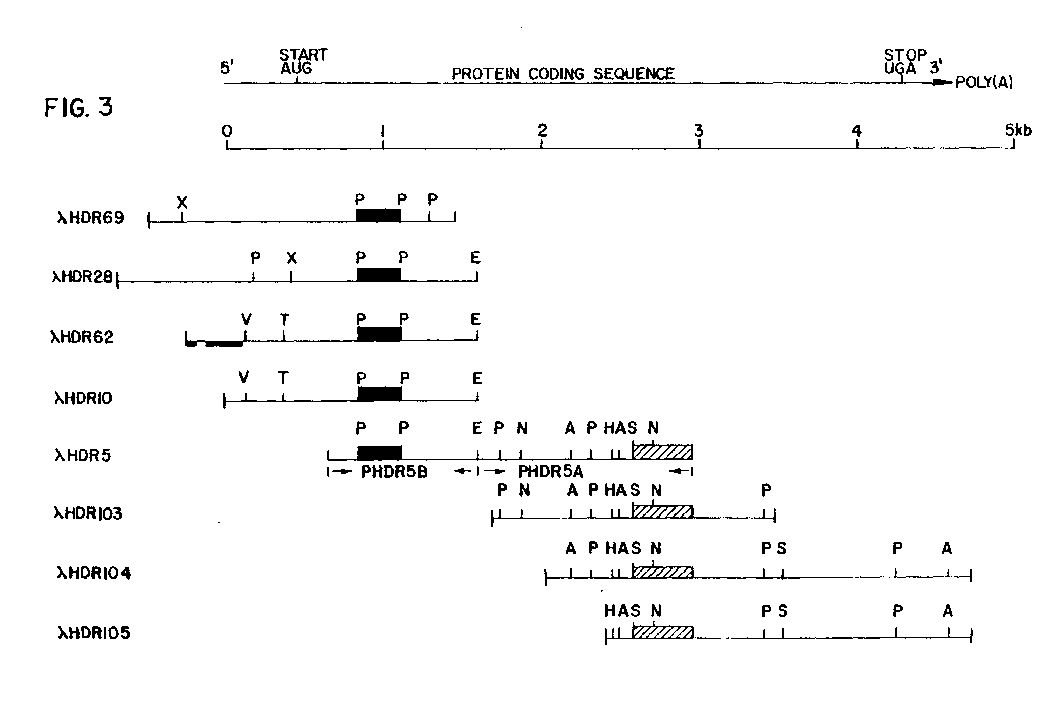

In order to isolate cDNA clones of the mdr1 gene, poly(A) + RNA was isolated as described in Chirgwin et al., Biochemistry, 18, 5294 (1979) and Aviv et al., Proc. Natl. Acad. Sci. (USA). 69, 1408 (1972) from the subline KB-C2.5, selected with colchicine. A cDNA library was constructed using the steps of synthesizing double-stranded cDNA, blunt ending, attachment of EcoRI linkers and insertion into the phage vector λgtII [Young and Davis, supra; Huynh et al., in: DNA Cloning Techniques: A Practical Approach, D. Glover, ed., IRL Press, Oxford, (1985)]. The cDNA library was screened by plaque hybridization (Benton et al., supra) with the pMDR1 probe. Approximately 120 positive clones were isolated. The inserts from five of these clones (λHDR5, λHDR10, λHDR28, λHDR62 and λHDR69) were re-cloned into plasmid vectors pGEM1 and pGEM4 (Promega Biotec). The partial restriction maps of these clones are shown in Fig. 3. DNA from λHDR5 was treated with EcoRI which generated two fragments, designated 5A and 5B. The fragments were subcloned into pGEM1 at its EcoRI site to give plasmids pHDR5A and pHDR5B which were deposited with the American Type Culture Collection, 12301 Parklawn Drive, Rockville, Maryland, on March 18, 1986, and which received the respective accession numbers ATCC 67040 and ATCC 67041. Similarly, λHDR10 was treated with EcoRI and cloned into the EcoRI site of pGEM1 to produce pHDR10 which was deposited with the American Type Culture Collection, 12301 Parklawn Drive, Rockville, Maryland, on March 18, 1986, as Deposit No. 67042.

To isolate the remaining portion of mdr1 cDNA, a fragment of the clone λHDR5, indicated with a striped bar in Fig. 3, was used to screen the same cDNA library. The inserts from three of the positive clones, designated λHDR103, λHDR104 and λHDR105, were re-cloned into the EcoRI sites of plasmid vectors pGEM1 and pGEM4, giving rise to plasmids designated pHDR103, pHDR104 and pHDR105, respectively. The plasmid pHDR104 was deposited with the American Type Culture Collection, 12301 Parklawn Drive, Rockville, Maryland, on July 16, 1986, as Deposit No. 67156.

A comparison of the restriction maps of individual clones indicates divergence in the cDNA structure among, for example, clones λHDR10, XHDR28 and λHDR69. The most highly conserved region among these clones is represented by a 270 bp Pvull fragment, which corresponds to the exon regions of pMDR1 and is indicated with a solid bar above the lines in Fig. 3. The variant sequences specific to clones XHDR62 and λHDR105 were detected by DNA sequencing, and they are shown as solid bars underneath the corresponding lines in Fig. 3. In Fig. 3, sites for digestion by corresponding restriction endonucleases are identified as follows: "A", AccI; "E", EcoRI; "H", HindIII; "N", XmnI; "P", Pvull; "S", Stul; "T", SstI; "V", Aval; and "X", Xbal.

The cDNA clones λHDR10, λHDR5 and λHDR104 were sequenced in their entirety using the methods of subcloning the inserts into an MI3 phage vector [Messing, Meth. Enyzmol., 101, 20, 1983], generating a series of overlapping deletion subclones [Henikoff, Gene, 28, 351, 1984] and determining their DNA sequence by the enzymatic chain-termination sequencing techniques [Sanger et al., supra]. A part of the cDNA sequence was determined by specific-primer-directed DNA sequencing [Strauss et al., Anal. Biochem., 154, 353 1986] using supercoiled plasmid DNA as a template [Zagursky et al., supra]. The overlapping regions of clones λHDR10, λHDR5 and λHDR104 were found to be identical, and therefore, these clones are assumed to represent different parts of the same cDNA. The combined cDNA sequence of clones λHDR10, λHDR5 and λHDR104 is shown in Table 5. This table also shows the amino acid sequence of mdr1 gene product, derived from the same cDNA sequence.

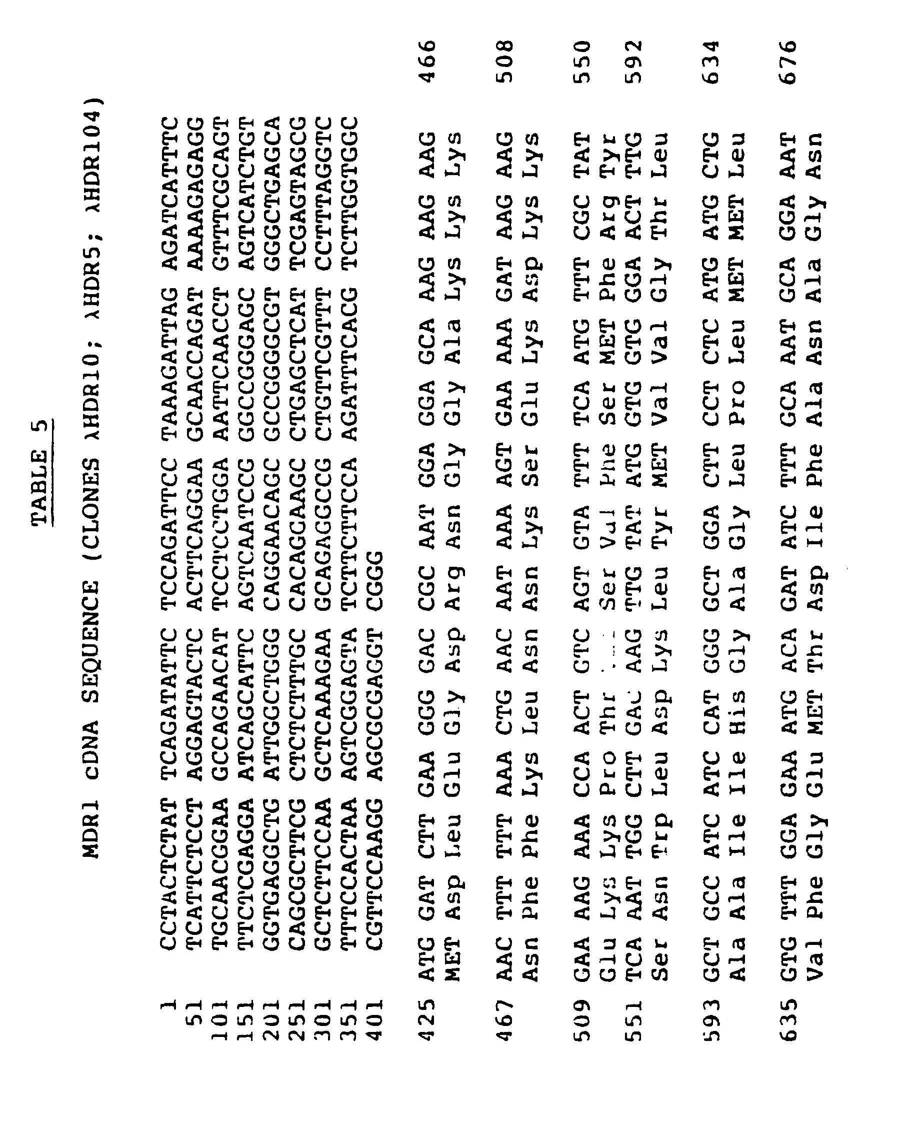

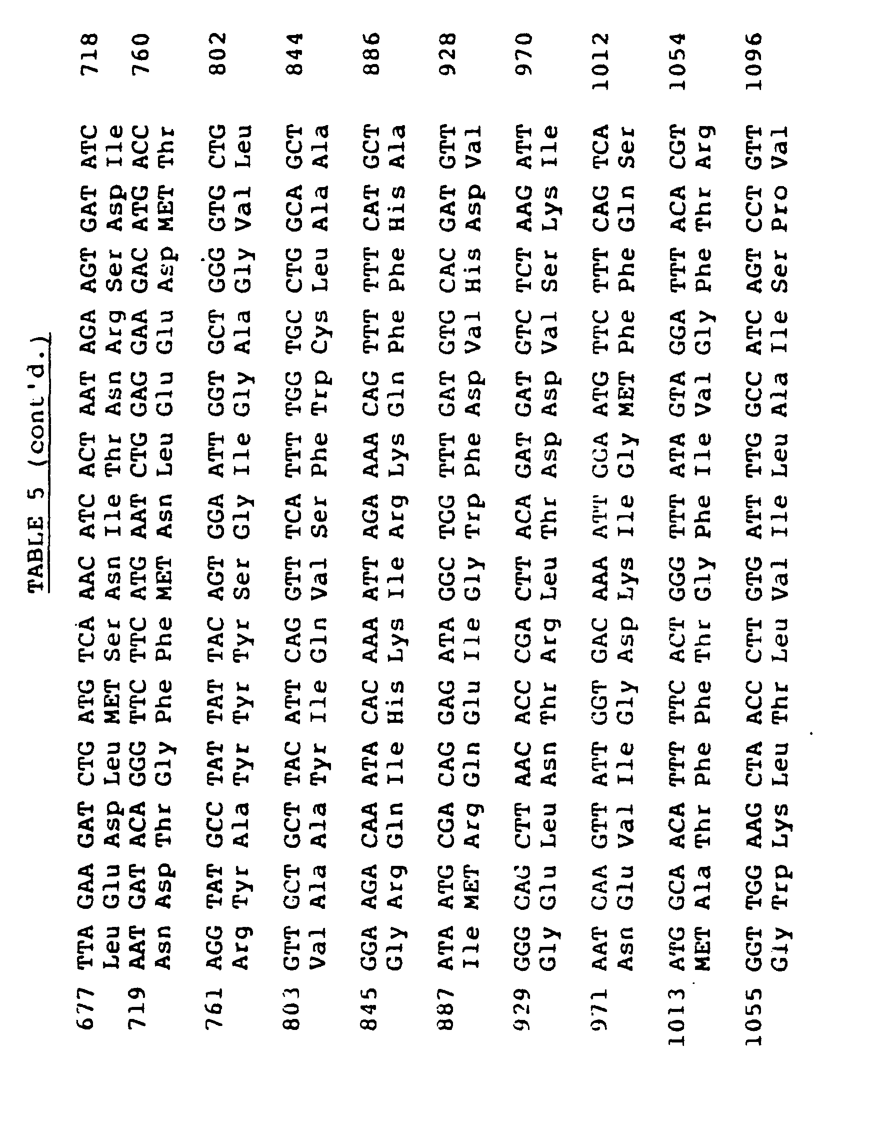

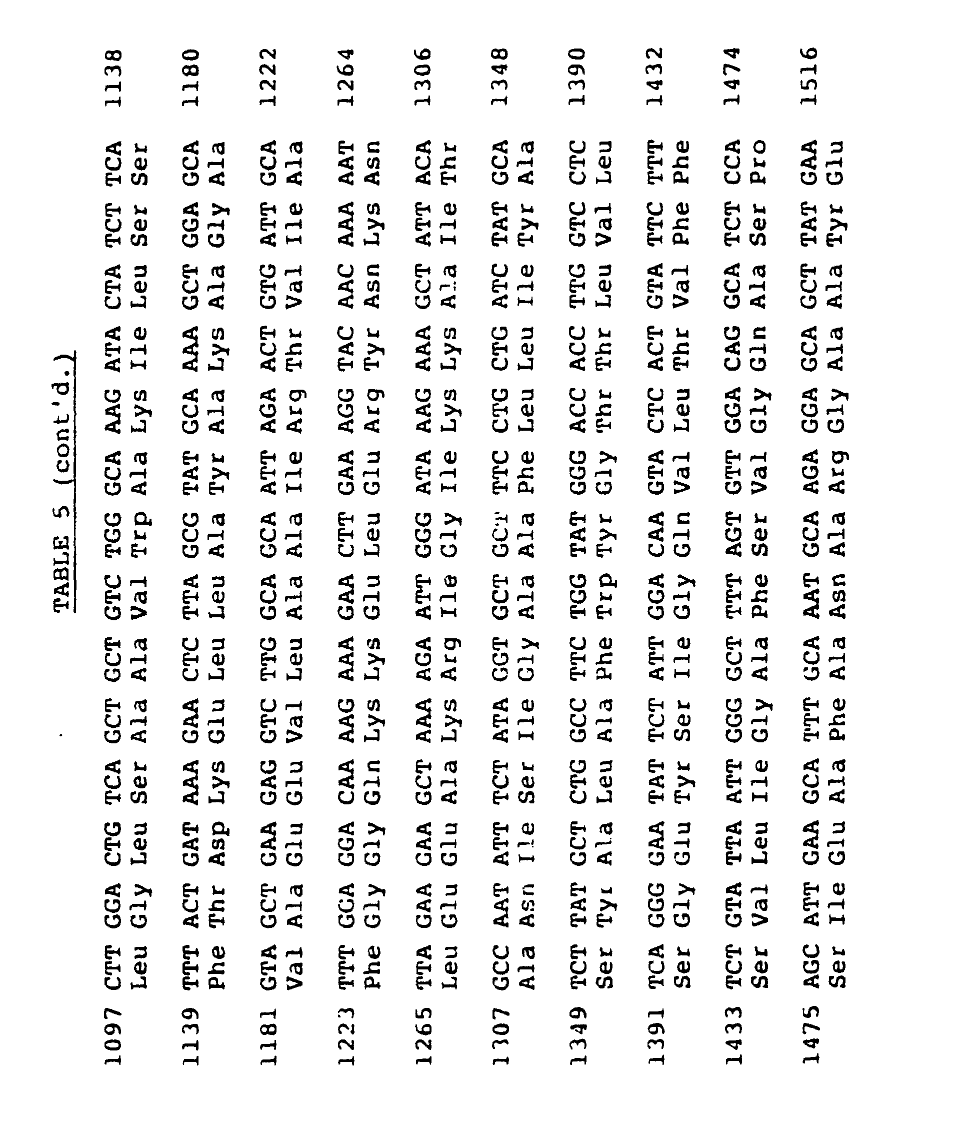

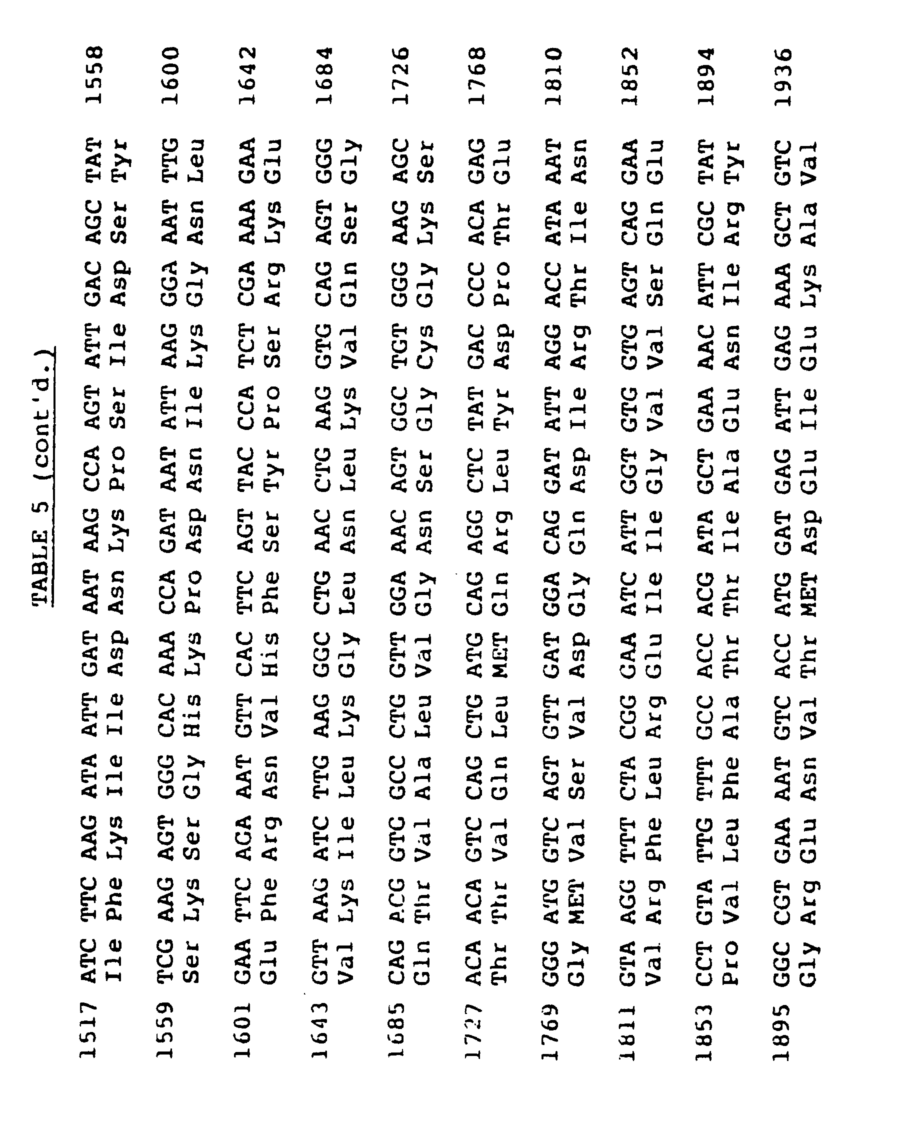

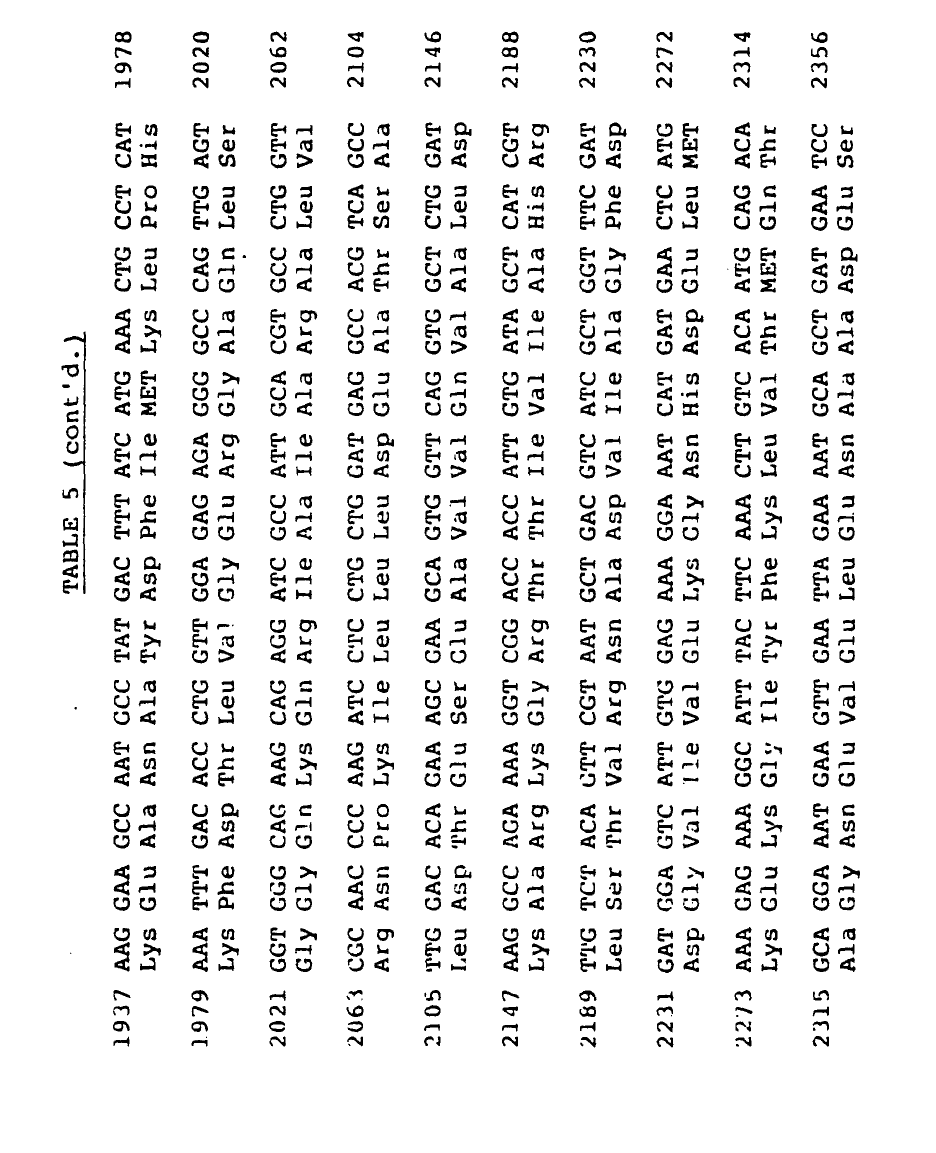

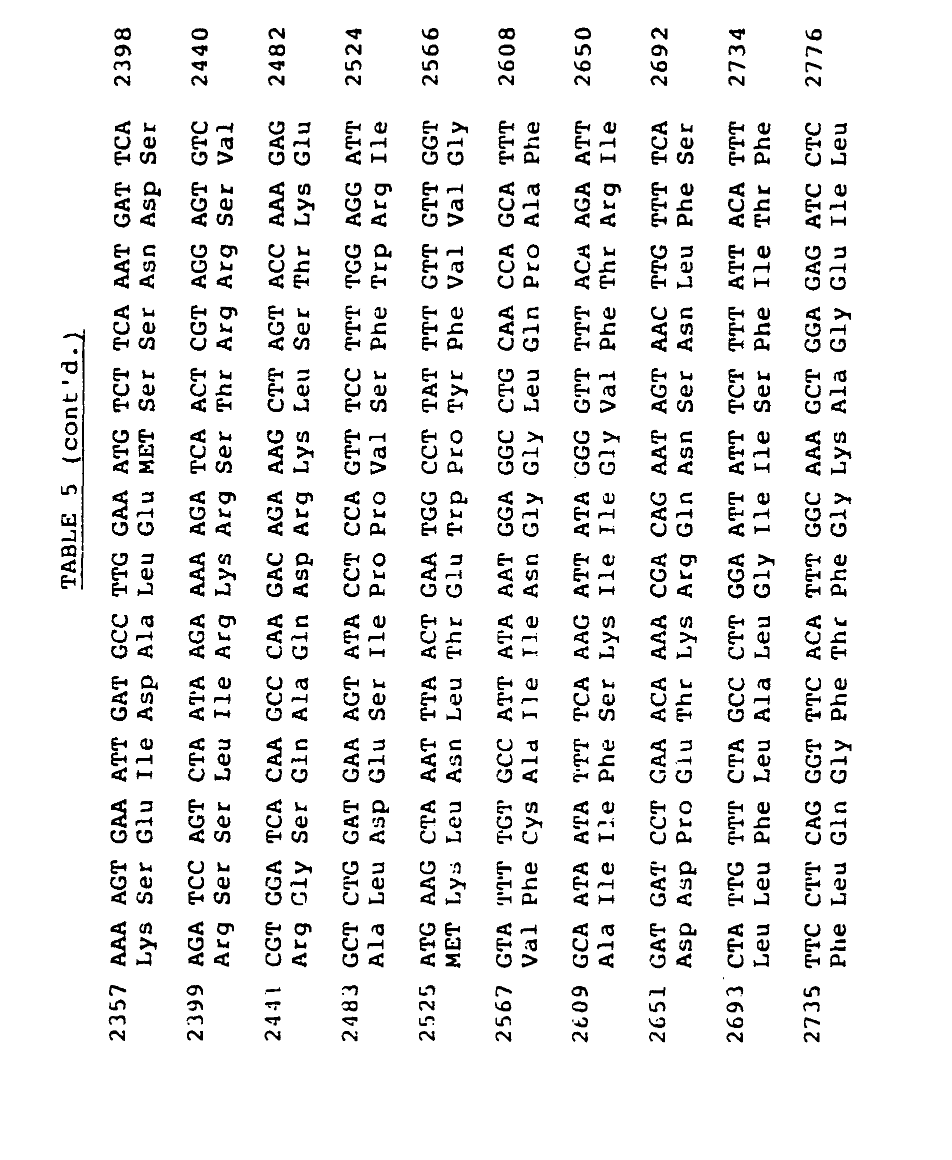

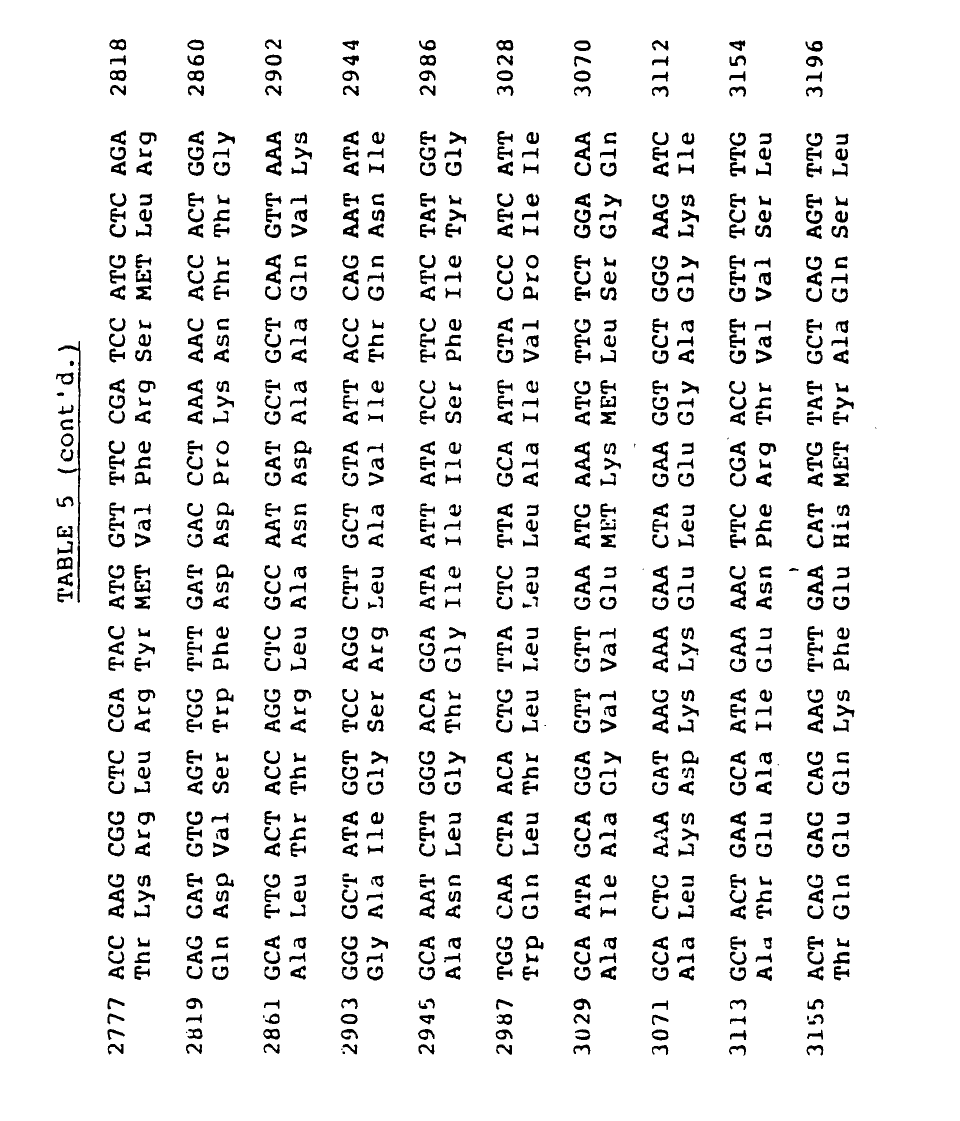

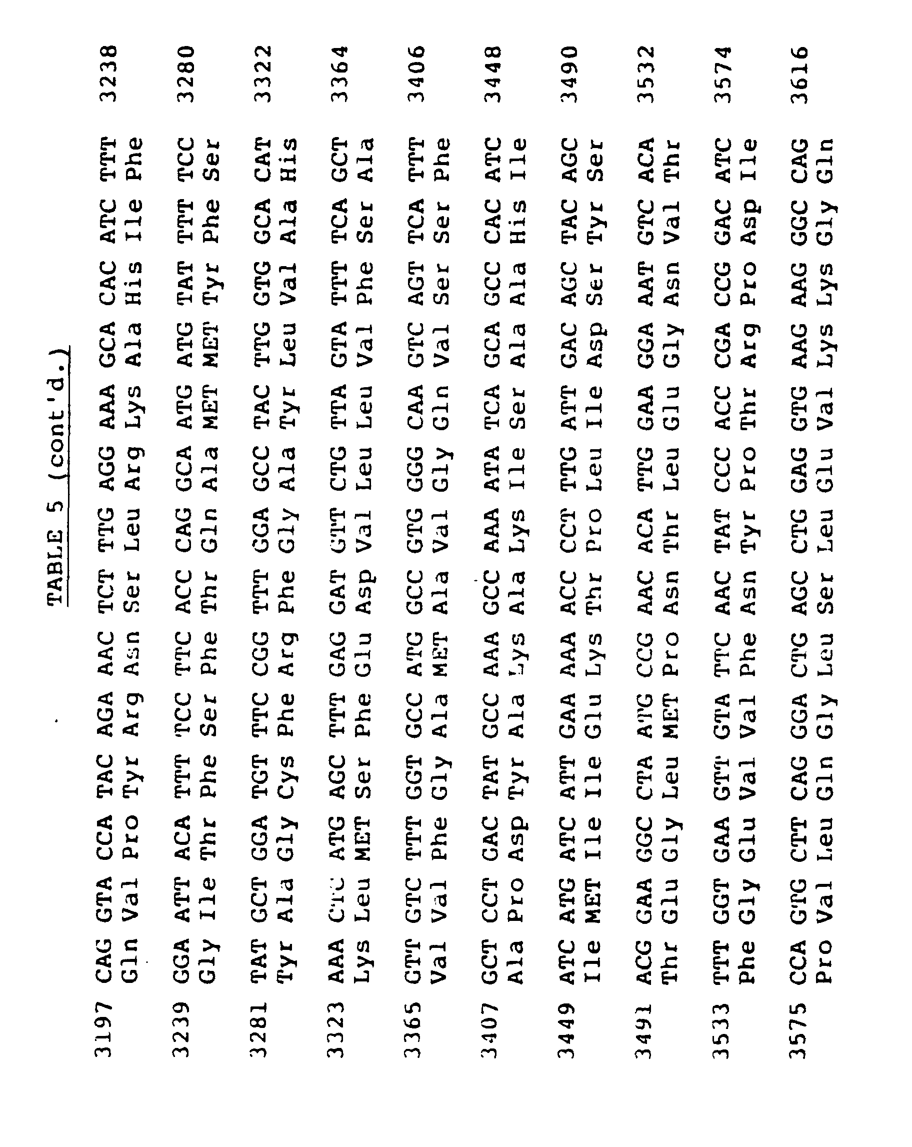

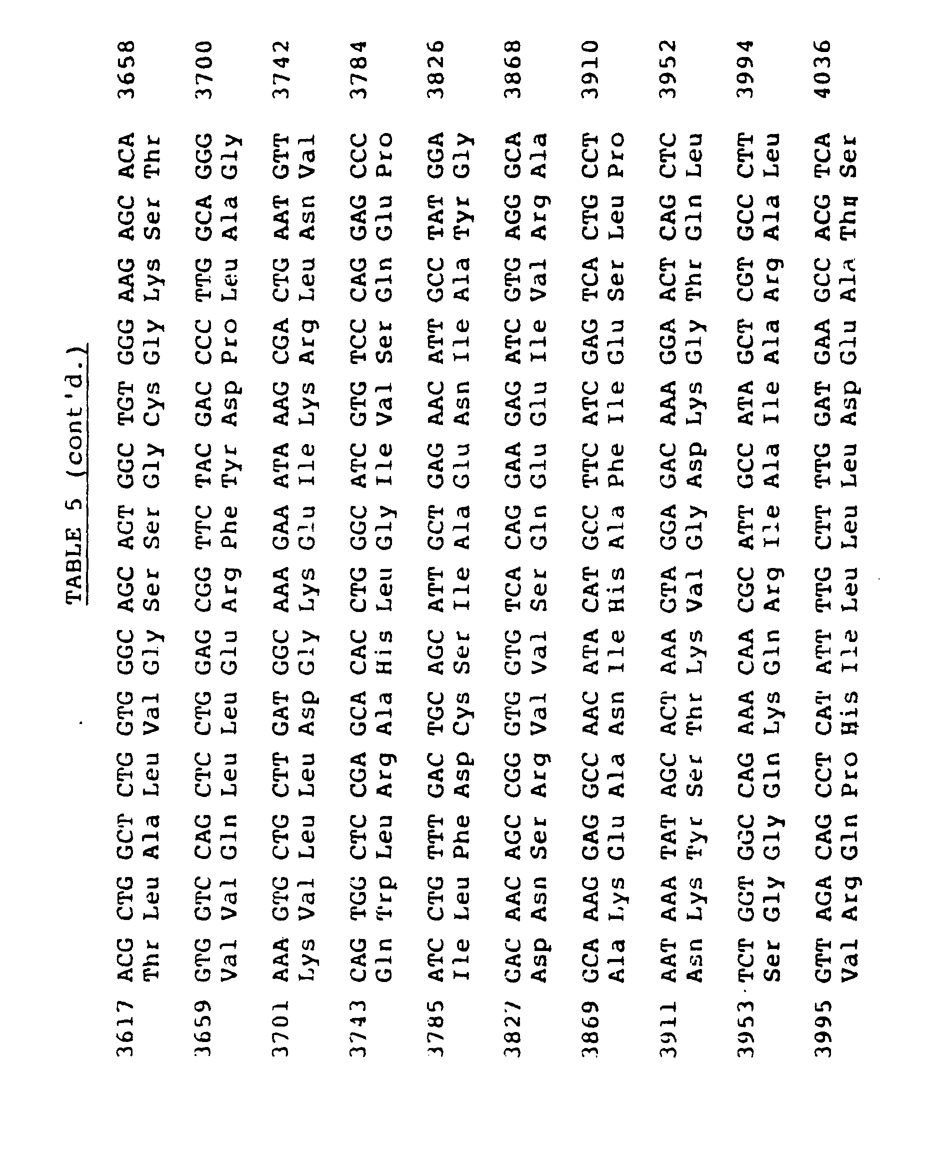

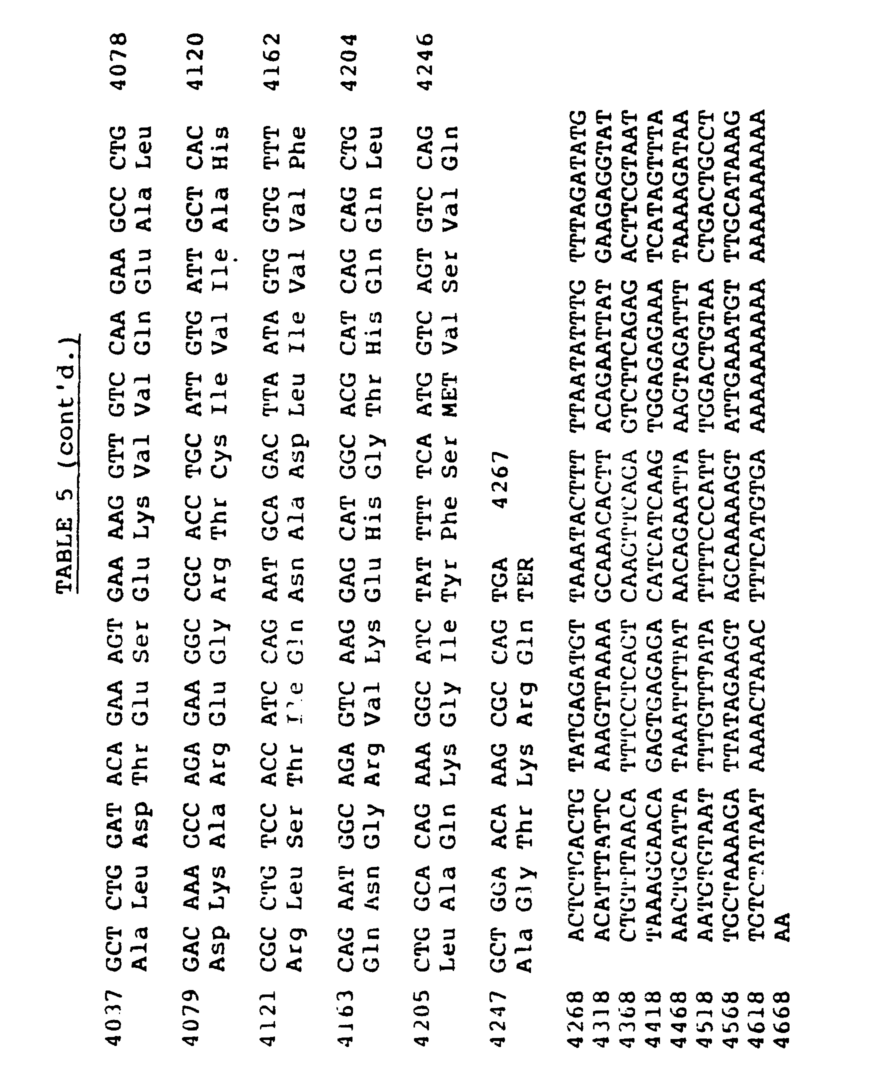

Analysis of the amino acid sequence presented in Table 5 indicates that the mdr1 gene product is likely to be a transmembrane protein. This protein may consist of two approximately equal parts, with a considerable sequence homology to each other, indicating that the mdr1 gene has likely evolved as a result of an internal duplication. Each half of the protein consists of a hydrophobic and a hydrophilic portion. Each of the hydrophobic portions includes six transmembrane domains, as determined by the algorithm of Eisenberg et al. [J. Mol. Biol., 179, 125-142 (1984)]. Both hydrophilic portions contain two regions that share a high level of amino acid homology with the ATP-binding sites of several known enzymes. The best homology has been observed with the ATP-binding sites of peripheral membrane components of bacterial periplasmic binding protein-dependent transport systems [Higgins et al., EMBO J., 4, 1033-1040, (1984)]. The presence of the transmembrane domains and potential glycosylation sites within the protein sequence is consistent with the mdr1 protein being related to the P-glycoprotein, which is described above.

Analysis of the DNA and protein sequence information presented in Table 5 by the algorithm of Eisenberg et al., supra, may be used to predict the protein regions that are located on the outside of the cell membrane. These protein regions may be produced either by chemical synthesis or by expression in the appropriate vector systems, and may be used to raise antibodies against cells that express the mdr1 gene product, as described in Example 11.

### Example 11

The recombinant plasmid pMDR1 as well as different individual fragments of recombinant plasmid pHDR4.4 or the latter plasmid as a whole, or cDNA clones λHDR5, λHDR10, λHDR62, λHDR28 and λHDR69, or other sequences according to the present invention, may be used as diagnostic tools for detection of human tumor cells resistant to chemotherapeutic drugs. These plasmids may be labeled directly with a radioactive isotope, according to the procedures of Rigby et al., Mol. Biol., 113, 237-251 (1977) or Feinberg et al., Anal. Biochem., 132, 6-13 (1983), for example. Alternatively, the plasmids may be labelled with a non-radioactive chemical tag, for example, according to the procedure in Leary et al., Proc. Natl. Acad. Sci. (USA), 80, 4045-4049 (1983). The plasmids may also be used to direct synthesis of labeled RNA probes [e.g., according to the procedure in Melton et al., Nucleic Acids Res., 12, 7035-7055 (1984)]. The labeled probes may then be used to detect the presence of homologous RNA sequences in tumor cells either by the Northern hybridization procedure [according to Thomas, Proc. Natl. Acad. Sci. (USA), 77, 5201-5205 (1980)] or by dot blot or slot blot hydridization [according to Kafatos et al., Nucleic Acids Res., 7, 1541-1552 (1979) and Brown et al., Mol. Cell. Biol., 3, 1097-1107 (1983)], or by in situ hybridization techniques [e.g., those according to the procedures of Brahic et al., Proc. Natl. Acad. Sci. (USA), 75, 6125-6129 (1978)]. It is anticipated that in situ hybridization will provide a particularly sensitive method for detection of a small number (1 in 1000 or fewer) of multidrug-resistant cells within a biopsy.

## Claims (Claims for the following Contracting State(s): BE, CH, DE, FE, IT, LI, LU, NL, SE)

1. An isolated nucleic acid that forms a stable hybrid at 65°C in a solution consisting of 0.1 x SSC, and 0.5% SDS with a polynucleotide having the sequence of nucleotides set forth in Table 5 or with the complement thereof.

2. An isolated nucleic acid as claimed in claim 1 that forms a stable hybrid at 65°C in a solution consisting of 0.1 x SSC, and 0.5% SDS with a polynucleotide having the sequence of nucleotides from nucleotide 1 to approximately nucleotide 3000 as set forth in Table 5 or with the complement thereof.

3. An isolated nucleic acid as claimed in claim 1 or claim 2, having the sequence of nucleotides 425-4267 as set forth in Table 5.

4. An isolated nucleic acid as claimed in claim 1 or claim 2 that is obtainable as DNA or cDNA from a human cell.

5. A nucleic acid probe, comprising a label and an isolated nucleic acid as claimed in any one of claims 1 to 4.

## Claims (Claims for the following Contracting State(s): AT)

1. A method of obtaining an isolated nucleic acid that forms a stable hybrid at 65°C in a solution consisting of 0.1 x SSC, and 0.5% SDS with a polynucleotide having the sequence of nucleotides set forth in Table 5 or with the complement thereof, which method comprises isolating the nucleic acid.

2. A method as claimed in claim 1, wherein the isolated nucleic acid as claimed forms a stable hybrid at 65°C in a solution consisting of 0.1 x SSC, and 0.5% SDS with a polynucleotide having the sequence of nucleotides from nucleotide 1 to approximately nucleotide 3000 as set forth in Table 5 or with the complement thereof.

3. A method as claimed in claim 1 or claim 2, wherein the isolated nucleic acid has the sequence of nucleotides 425-4267 as set forth in Table 5.

4. A method as claimed in claim 1 or claim 2, wherein the isolated nucleic acid is obtainable as DNA or cDNA from a human cell.

5. A method of obtaining a nucleic acid probe comprising a label and an isolated nucleic acid, which comprises deriving said probe from an isolated nucleic acid as claimed in any one of claims 1 to 4.

## Patentansprüche (Patentansprüche für folgende(n) Vertragsstaat(en): BE, CH, DE, FR, GB, IT, LI, LU, NL, SE)

1. Isolierte Nukleinsäure, die bei 65°C in einer Lösung, die aus 0,1 x SSC und 0,5 % SDS besteht, ein stabiles Hybrid bildet mit einem Polynukleotid mit der Sequenz von Nukleotiden, die in Tabelle 5 angegeben ist, oder mit dem Komplement davon.

2. Isolierte Nukleinsäure nach Anspruch 1, die bei 65°C in einer Lösung, die aus 0,1 x SSC und 0,5 % SDS besteht, ein stabiles Hybrid bildet mit einem Polynukleotid mit der Sequenz von Nukleotiden von Nukleotid 1 bis ungefähr Nukleotid 3000, wie angegeben in Tabelle 5, oder mit dem Komplement davon.

3. Isolierte Nukleinsäure nach Anspruch 1 oder Anspruch 2, mit der Sequenz der Nukleotide 425 bis 4267, wie angegeben in Tabelle 5.

4. Isolierte Nukleinsäure nach Anspruch 1 oder Anspruch 2, die als DNA oder cDNA aus einer menschlichen Zelle erhältlich ist.

5. Nukleinsäuresonde, die eine Markierung und eine isolierte Nukleinsäure nach einem der Ansprüche 1 bis 4 umfaßt.

## Patentansprüche (Patentansprüche für folgende(n) Vertragsstaat(en): AT)

1. Verfahren zum Erhalten einer isolierten Nukleinsäure, die bei 65°C in einer Lösung, die aus 0,1 x SSC und 0,5 % SDS besteht, ein stabiles Hybrid bildet mit einem Polynukleotid mit der Sequenz von Nukleotiden, die in Tabelle 5 angegeben ist, oder mit dem Komplement davon, wobei das Verfahren das Isolieren der Nukleinsäure umfaßt.

2. Verfahren nach Anspruch 1, **dadurch gekennzeichnet, daß** die isolierte Nukleinsäure, wie beansprucht, bei 65°C in einer Lösung, die aus 0,1 x SSC und 0,5 SDS besteht, ein stabiles Hybrid bildet mit einem Polynukleotid mit der Sequenz von Polynukleotiden von Nukleotid 1 bis ungefähr Nukleotid 3000, wie angegeben in Tabelle 5, oder mit dem Komplement davon.

3. Verfahren nach Anspruch 1 oder Anspruch 2, **dadurch gekennzeichnet, daß** die isolierte Nukleinsäure die Sequenz der Nukleotide 425 bis 4267, wie angegeben in Tabelle 5, aufweist.

4. Verfahren nach Anspruch 1 oder Anspruch 2, **dadurch gekennzeichnet, daß** die isolierte Nukleinsäure als DNA oder cDNA aus einer menschlichen Zelle erhältlich ist.

5. Verfahren zum Erhalten einer Nukleinsäuresonde, die eine Markierung und eine isolierte Nukleinsäure umfaßt, welches das Gewinnen besagter Sonde aus einer isolierten Nukleinsäure, wie beansprucht in einem der Ansprüche 1 bis 4, umfaßt.

## Revendications (Revendications pour l'(les) Etat(s) contractant(s) suivant(s): BE, CH, DE, FR, GB, IT, LI, LU, NL, SE)

1. Acide nucléique isolé qui forme un hybride stable à 65°C dans une solution consistant en 0,1 x SSC et 0,5% SDS avec un polynucléotide ayant la séquence de nucléotides indiquée au Tableau 5 ou avec son complément.

2. Acide nucléique isolé selon la revendication 1 qui forme un hybride stable à 65°C dans une solution consistant en 0,1 x SSC et 0,5% SDS avec un polynucléotide ayant la séquence des nucléotides à partir du nucléotide 1 à peu près jusqu'au nucléotide 3000 comme indiqué au Tableau 5 ou avec son complément.

3. Acide nucléique isolé selon la revendication 1 ou la revendication 2, ayant la séquence de nucléotides 425-4267 telle qu'indiquée au Tableau 5.

4. Acide nucléique isolé selon la revendication 1 ou la revendication 2 qui peut être obtenu sous la forme d'ADN ou d'ADNc d'une cellule humaine.

5. Sonde d'acide nucléique comprenant un marqueur et un acide nucléique isolé selon l'une quelconque des revendications 1 à 4.

## Revendications (Revendications pour l'(les) Etat(s) contractant(s) suivant(s): AT)

1. Méthode d'obtention d'un acide nucléique isolé qui forme un hybride stable à 65°C dans une solution consistant en 0,1 x SSC, et 0,5% SDS avec un polynucléotide ayant la séquence de nucléotides indiquée au Tableau 5 ou avec son complément, laquelle méthode comprend l'isolement de l'acide nucléique.

2. Méthode selon la revendication 1, où l'acide nucléique isolé tel que revendiqué forme un hybride stable à 65°C dans une solution consistant en 0,1 x SSC et 0,5% SDS avec un polynucléotide ayant la séquence des nucléotides du nucléotide 1 à peu près jusqu'au nucléotide 3000 telle qu'indiquée au Tableau 5 ou avec son complément.

3. Méthode selon la revendication 1 ou la revendication 2 où l'acide nucléique isolé a la séquence des nucléotides 425-4267 telle qu'indiquée au Tableau 5.

4. Méthode selon la revendication 1 ou la revendication 2 où l'acide nucléique isolé peut être obtenu sous la forme d'ADN ou d'ADNc d'une cellule humaine.

5. Méthode d'obtention d'une sonde d'acide nucléique comprenant un marqueur et un acide nucléique isolé, qui consiste à dériver ladite sonde d'un acide nucléique isolé tel que revendiqué dans l'une quelconque des revendications 1 à 4.
